# EUROPÄISCHE PATENTSCHRIFT

(11) **EP 3 997 444 B1**
(45) Veröffentlichungstag und Bekanntmachung des Hinweises auf die Patenterteilung: **14.01.2026**
(21) Anmeldenummer: 20750569.4
(22) Anmeldetag: 06.07.2020
(51) Int. Cl.: G01N 21/64, G01N 15/14, G01N 15/1434, G01N 15/00

(54) **VORRICHTUNG UND VERFAHREN ZUR BESTIMMUNG DER FLUORESZENZ UND DER ANZAHL VON ANTIKÖRPERN AUF EXOSOMEN MIT REDUZIERUNG DER INTENSITÄTSMINDERUNG DES FLUORESZENZFARBSTOFFS DURCH LASERLICHT**
DEVICE AND METHOD FOR DETERMINING THE FLUORESCENCE AND THE NUMBER OF ANTIBODIES ON EXOSOMES WITH MITIGATION OF THE INTENSITY REDUCTION OF THE FLUORESCENT DYE BY LASER LIGHT
DISPOSITIF ET PROCÉDÉ POUR LA DÉTERMINATION DE LA FLUORESCENCE ET DU NOMBRE D'ANTICORPS SUR DES EXOSOMES AVEC DIMINUTION DE LA RÉDUCTION DE L'INTENSITÉ DU COLORANT FLUORESCENT PAR LA LUMIÈRE LASER

(30) Priorität: 11.07.2019 DE 102019004870
(43) Veröffentlichungstag der Anmeldung: 18.05.2022
(73) Patentinhaber: Particle Metrix GmbH, 82266 Inning a. Ammersee (DE)
(72) Erfinder: WACHERNIG, Hanno, 86911 Diessen am Ammersee (DE); HELMBRECHT, Clemens, 82229 Seefeld (DE); SCHIFFMANN, Jens, 82237 Wörthsee (DE)
(74) Vertreter: Weickmann & Weickmann PartmbB
(86) Internationale Anmeldenummer: PCT/DE2020/000149
(87) Internationale Veröffentlichungsnummer: WO 2021/004563

(56) Entgegenhaltungen:
- EP-A2- 2 594 981
- DE-U1- 202018 005 287
- C HELMBRECHT ET AL: "High efficiency quantification of fluorescent labeled EVs with F-NTA", 17 May 2017 (2017-05-17), XP055714860, Retrieved from the Internet <URL:https://www.particle-metrix.de/fileadmin/pdf_technologien/Poster_A0_Fluorescence_EN.pdf> [retrieved on 20200714]

## Beschreibung

Die Anmeldung betrifft eine Vorrichtung zur Bestimmung der Fluoreszenz und der Anzahl von Antikörpern auf Exosomen.

Aus der DE 20 2018 005 287 U1 ist eine Vorrichtung zur Bestimmung der Fluoreszenz und der Anzahl von Antikörpern auf Exosomen bekannt.

Vesikel in der Biologie sind intrazelluläre, sehr kleine, rundliche bis ovale Bläschen, die von einer einfachen oder doppelten Membran oder einer netzartigen Hülle aus Proteinen umgeben sind. Die Vesikel bilden eigene Zellkompartimente, in denen unterschiedliche zelluläre Prozesse ablaufen. Ihre Größe beträgt etwa einen Mikrometer. Vesikel sind für den Transport vieler Stoffe in der Zelle verantwortlich,

Die Mechanismen, die zur Entstehung von extrazellulären Vesikeln führen, sind bisher noch nicht vollständig geklärt. Man unterscheidet aufgrund ihrer Herkunft oder Größe drei Arten von extrazellulären Vesikeln.

Exosomen sind hierbei kleine Vesikel mit einer Größe von ca. 50 bis 150 nm.

Gemäß dem Anspruch 1 der oben genannten Druckschrift handelt es sich um eine Vorrichtung mit den folgenden Merkmalen:
a) die Strahlen von mehreren verschiedenen Lasern (1,2,3,4) werden jeweils mittels eines gesonderten Sammelprismas (14) separat auf einen Strahlengang (21) in eine Messzelle (22) mit einer Partikel enthaltenden Probe (9) gerichtet, wobei die Fokussierung des Laserstrahls (21) in Wechselwirkung mit der Probe (9) das Zentrum eines konvergenten Strahlenbündels, bestehend aus Licht aus der Fluoreszenzebene (5) und der Streulichtebene (8) bildet, das nach Durchgang durch eine Flüssigkeitslinse mit einer Optik - Steuerung (18) in einer Videokamera (15) registriert wird,
b) der konvergente Strahlengang tritt durch ein Farbfilter (16), das mittels eines Wechselrades (17) und einer Steuerung (26) bewegt wird,
c) ein Display (19) mit einem Touchscreen (19) und eine Gesamt - Steuerung (20) mit einem Partikel - Tracking - Programm dienen einer Videokamera (15).

Mehrere Messungen hintereinander mit der gleichen Probe, um ein statistisch gutes Ergebnis zu erzielen, kann bei dem geschilderten Stand der Technik in der DE 20 2018 005 287 U1 nicht durchgeführt werden, da hierbei der verwendete Fluoreszenzfarbstoff durch die lange Lasereinwirkzeit zu stark ausbleicht und eine Erneuerung der Probe in der Messzelle nach einer Messung notwendig macht. Würde die Einwirkzeit der Laser auf den Laserfarbstoff (Fluorochrome) verkürzt werden, könnte das Ausbleichen reduziert werden, und mehrere Messungen mit ein und derselben Probe könnten ermöglicht werden.

Der vorliegenden Anmeldung liegt deshalb die Aufgabe zugrunde, die Einwirkzeit des Laserlichts auf die Fluorochrome (Fluoreszenzfarbstoff) zu verkürzen.

Die Aufgabe wird durch die unabhängigen Ansprüche gelöst.

Eine erfindungsgemäße Vorrichtung umfasst die Merkmale von Anspruch 1.

Es sind Mittel zur Speicherung von verschiedenen Messpunkten (26) unterschiedlicher verschiedenfarbiger Laser in einer Messzelle (17) an bestimmten Messpositionen vorgesehen, wobei die Fokussierung des jeweiligen Laserstrahls (24) in Wechselwirkung mit der Probenflüssigkeit (18) als Zentrum eines konvergenten Strahlenbündels in einer Videokamera der Vorrichtung registriert wird.

Eine Wechselvorrichtung (7), mit einem Multi-Notch-Filter (6) und einem Quarzglas 16 dient zur Einstellung und Sicherung der gleichen optischen Weglängen für das Fluoreszenzlicht und das Laserstreulicht im konvergenten Strahlengang zwischen der Flüssigkeitslinse (15) und den Messpunkten 26 in der Messzelle (17).

Ein Display (9) mit einem Touchscreen und eine Gesamt-Steuerung (10) mit einem Partikel-Tracking-Programm dienen der Bedienung der Videokamera (13), wobei die Videokamera (13) vorzugsweise einen Graphen-basierten Lichtsensor (12) mit einer zugehörigen Steuerung (11) für den Lichtsensor (12) aufweist und schnelle Schrittmotoren und spielfreie Präzisions-Verfahrschlitten aufweist, und die Kameras bevorzugt ein CMOS oder ein eCCD ist.

Das erfindungsgemäße Verfahren ist in Anspruch 4 beschrieben.

Die Messzelle (17) wird mit Prüfflüssigkeit befüllt, zur Erfassung der verschiedenen Positionen der verwendeten Laser, deren Fokussierung und Speicherung.

Ein Tausch der Kalibrierflüssigkeit gegen Probenflüssigkeit mit zu messenden Exosomen und eine Prüfung auf Blasenfreiheit in der Zelle (17) finden statt. Alle Laser werden auf die erste Position zurückgefahren,

Die verwendeten Laser werden nacheinander auf ihren abgespeicherten Fokuspunkten angefahren und beim Beginn der Analyse eingeschaltet, die Bilder werden von dem Lichtsensor (12) aufgenommen und die entsprechenden Daten werden an die Mustererkennung weiter geleitet.

Die Figuren der Anmeldung haben folgenden Inhalt.
- Fig.1:: eine Darstellung einer besonderen NTA Nanopartikeltracking - Methode.
- Fig. 2:: eine Darstellung der Messzelle,
- Fig.3:: eine Darstellung der Abnahme der Intensität des Farbstoffs nach Lasereinwirkung in Sekunden
- Fig.4:: eine Darstellung der Messpositionen und der Fokus-Punkte der Laser (Messpunkte)

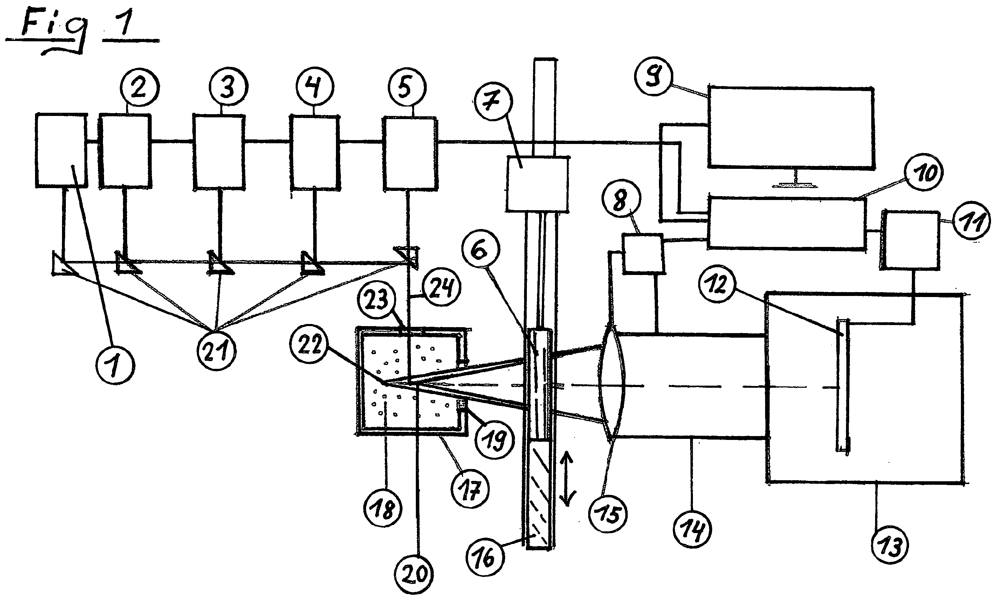

Die Fig. 1 entspricht in wesentlichen Teilen der Darstellung der Fig.1 aus der DE 20 2018 005 287 U1.

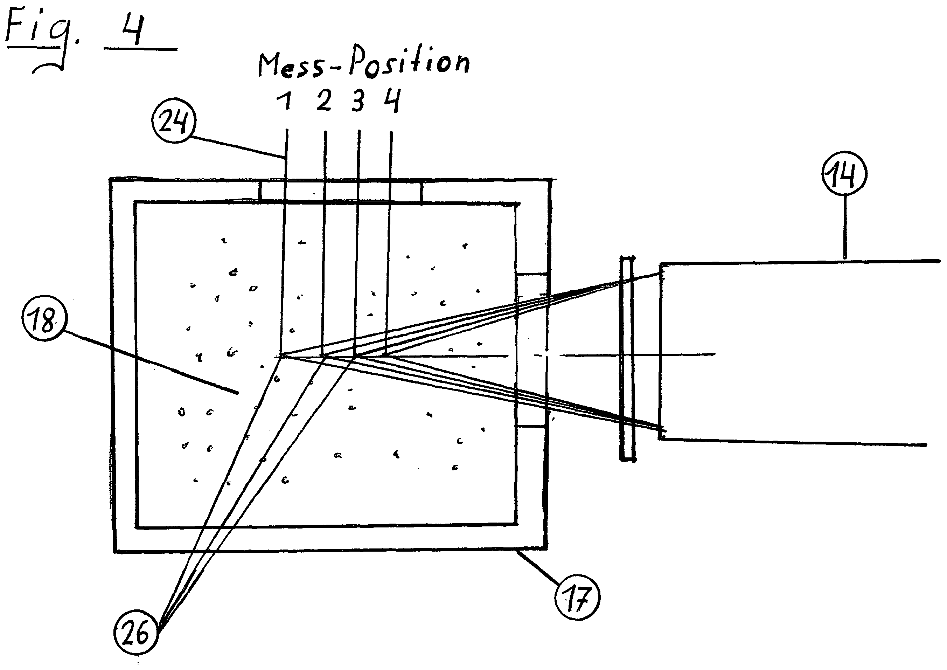

Die Anzahl der Laser beträgt fünf anstelle von vier im St.d.T. Das FlüssigkeitsFilter 6 beim St.d.T. ist ersetzt durch ein Multi-Notch-Filter 6 mit einer zugehörigen Wechselvorrichtung 7, für das Multi-Notch-Filter 6 und ein Quarzglas 16. Das Quarzglas 16 dient zur Einstellung und Sicherung der gleichen optischen Weglängen für das Fluoreszenzlicht und das Laserstreulicht im konvergenten Strahlengang durch das Objektiv zum Lichtsensor 12 bei der Justierung der Messpunkte 26 (siehe Figur 4) in der Messzelle 17. Das Quarzglas simuliert die gleichen optischen Weglängen wie das Notchfilter.

Die Videokamera 15 beim St.d.T hat hier die Nummer 13 und weist einen Graphen-basierten Lichtsensor 12 auf, mit einer Steuerung 11 für den Lichtsensor 12, und steuert mittels der Kameraoptik 14 über eine Optik-Steuerung 8 eine Flüssigkeitslinse 15, die wiederum über das Notchfilter 6 in der Fluoreszenzebene 20 in der Probenflüssigkeit 18 der Messzelle 17 und das Optikdurchgangsfenster 19 in der Messzelle 17 auf einen Strahlengang 24 eines der Laser 1 bis 5 und eines der Sammelprismen 21 trifft.

Die Gesamtsteuerung 10 entspricht der Gesamtsteuerung 20 beim St.d.T., wobei das Display 9 dem entsprechenden Display 19 entspricht.

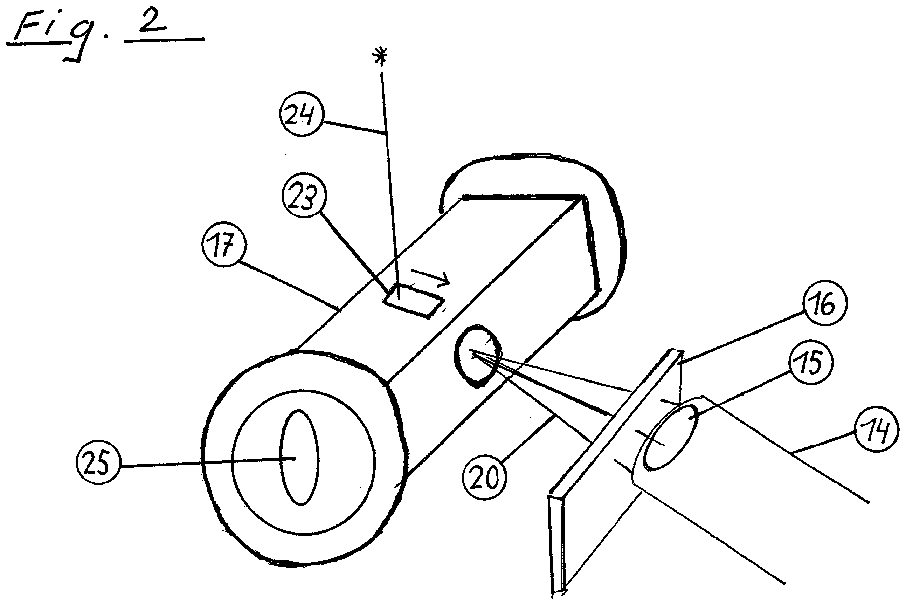

Die Darstellung der Messzelle 17 in der Fig. 2 zeigt senkrecht von oben den auch in der Fig.1 dargestellten Laser-Strahlengang, 24 der durch das Laser-Durchgangfenster 23 tritt, wobei seitwärts der im jeweiligen Messpunkt 26 spitz zulaufende Strahl zu sehen ist, der aus der Messzelle 17 durch das Quarzglas 16 und die Flüssigkeitslinse 5 und in die Kameraoptik 14 tritt.

Die Befüllöffnung 25 der Messzelle 17 ist auf der Vorderseite zu sehen.

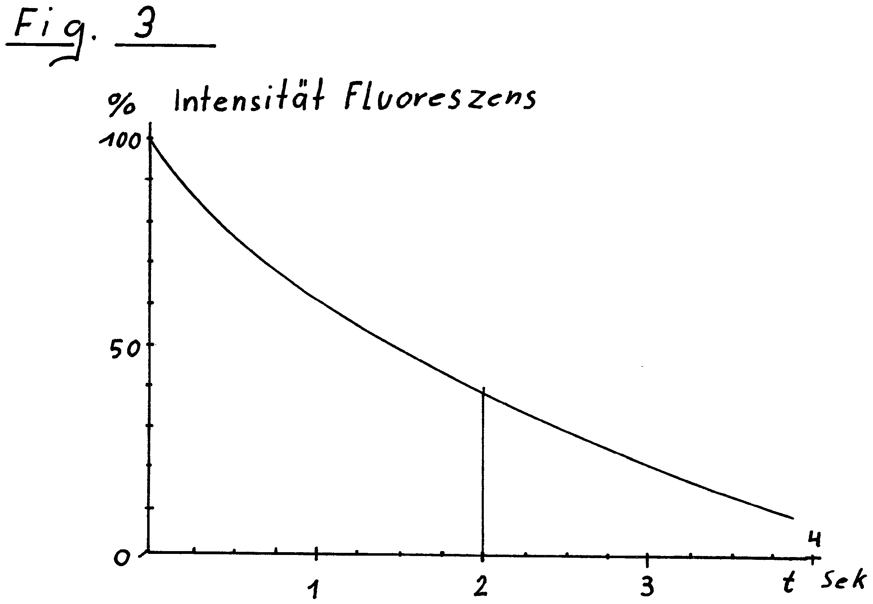

Die Abnahme der Intensität des Farbstoffes nach der Lasereinwirkung in Sekunden ist prozentual der Fig. 3 gut zu entnehmen. Das Laserlicht soll maximal 2 Sekunden auf die Fluoreszenz am Messpunkt 26 einwirken. Danach wird die Intensität des Fluoreszenzfarbstoffs zu schwach und liefert keine brauchbaren Messergebnisse.

Die Darstellung der Messpunkte 26 für die Fokus-Punkte (der Laserstreulichtebene und der Fluoreszenzlichtebene) der Optik zum Strahlengang der Laser 24 sind in den gezeigten verschiedenen Messpositionen 1 bis 4 der Fig. 4 zu entnehmen. Die Kameraoptik 14 ist hier ebenfalls dargestellt.

Beispielhaft sind die in der Fig.4 gezeigten 4 Positionen mit der Anordnung nach der Fig. 1 im Folgenden durchgeführt.

Die Messzelle 17 wird mit Prüfflüssigkeit (auch als Kalibrierflüssigkeit bezeichnet) gefüllt, wobei diese Prüfflüssigkeit Polystyrol-Partikel oder zertifizierte Exosomen-Standards enthält anstelle von der Exosomenprobe

**Im** Folgenden werden die einzelnen Laser in den jeweiligen Messpunkt 26 der Messzelle 17 geführt (siehe Figur 4) und gespeichert.

Das heißt, alle 5 Laser werden jeweils in die Messposition 1 geführt, die optischen Weglängen werden ermittelt, es wird fokussiert und dann gespeichert. Weiter werden dann alle 5 Laser in die nächste Messposition geführt, fokussiert und dann wird gespeichert.

Dasselbe geschieht mit der Position 3 und der Position 4 und allen 5 Lasern.

Dann werden die Laser ausgeschaltet.

Als nächster Schritt wird die Kalibrierflüssigkeit ausgetauscht gegen eine Probenflüssigkeit mit Exosomen in der Messzelle.

Als nächster Schritt wird das Notch-Filter 6 zwischen die Flüssigkeitslinse 15 und die Messzelle 17 geschoben und in den konvergierenden Strahlengang eingefahren, wobei das Quarzglas16 in Verbindung mit der Vorrichtung 7 zur Fixierung dient.

Die eigentliche Messung beginnt auf Messposition 1 damit, dass die fünf Laser nacheinander auf ihren abgespeicherten Fokuspunkten durchgeschaltet werden, und die Bilder von dem Lichtsensor aufgenommen werden, und die Daten an die Mustererkennung weiter geleitet werden. Die benötigte Zeit hierzu beträgt unter zwei Sekunden. Danach werden die Laser ausgeschaltet.

Weiter fahren die Laser auf Messposition 2. Es folgt die Messung wie im vorherigen Schritt. Danach werden die Laser ausgeschaltet.

Die Laser fahren weiter auf die Messposition 3. Weiter erfolgt die Messung wie im vorherigen Schritt. Danach werden die Laser ausgeschaltet.

Die Laser fahren auf die Messposition 4 . Es erfolgt die Auswertung der Daten.

Die Partikel können an verschiedenen Stellen in der Messzelle unterschiedlich hohe oder niedrige Konzentrationen von Partikeln bilden, zum Beispiel durch die Befüllung. Dadurch sind mehrere Messpositionen in der Messzelle nötig, um eine gute Qualität und eine hohe statistische Sicherheit der Messung zu erhalten.

**In** den neu angefahrenen Messpositionen in der Messzelle sind die Fluoreszenzen auf den Antikörpern noch frisch und unverbraucht.

Durch das Ausschalten der Laser beim Verfahren auf die nächsten Messposition wird die Fluoreszenz weiter geschont, da kein Laserlicht die Farbstoffe ausbleichen kann Dies ist nur durch das vorherige Abspeichern der einzelnen Messpositionen und Fokuspunkte und das hoch präzise schnelle Wiederanfahren der Positionen möglich (schnelle Schrittmotoren, spielfreier Präzisionsverfahrschlitten bei den Lasern und der Flüssigkeitslinse im Objektiv).

Das Multibandpassfilter 6 (Multi-Notch-Filter) ermöglicht das schnelle Durchschalten der einzelnen Laser, Dadurch ergibt sich eine Zeitersparnis für die Schonung der Fluoreszenz, da kein Filterwechsel nötig ist.

Es können durch die gezeigte schonende Messmethode bei einer und derselben Exosomen-Fluoreszenz-Probe nicht nur, wie in unserem Beispiel vier sondern bis zu 100 und mehr Messpositionen mit den Messpunkten 26 angefahren und mit fünf Lasern gemessen werden. Das gewährleistet eine sehr hohe Qualität der Messung.

Bei der Kamera muss ein hochempfindlicher sCMOS oder ein eCCD oder ein Graphen-Lichtsensor verwendet werden, da das Multi-Bandpassfilter 6 (Multi-Notchfilter) die Lichtintensität senkt.

Die Methode erfordert eine komplexe Steuerung, der beschriebenen Verfahrens- und Bewegungsabläufe, durch ein spezielles Steuerungs- und Analyseprogramm

### Bezugszeichenliste

- 1: Laser 375 nm
- 2: Laser (violett = 405 nm)
- 3: Laser (blau = 488 nm)
- 4: Laser (grün = 520 nm)
- 5: Laser (rot = 640 nm)
- 6: Notchfilter (Multibandpassfilter)
- 7: Wechsel - Vorrichtung für das Notchfilter und das Quarzglas
- 8: Optik - Steuerung
- 9: Display mit Touchscreen
- 10: Gesamt-Steuerung mit Partikel-Tracking-Programm
- 11: Steuerung für Lichtsensor
- 12: Graphen-basierter Lichtsensor
- 13: Detektor bzw. Videokamera eCCD, sCMOS
- 14: Kameraoptik
- 15: Flüssigkeitslinse mit einstellbarem Fokus
- 16: Quarzglas
- 17: Messzelle
- 18: Probe
- 19: Optik - Durchgangsfenster der Messzelle 17
- 20: Fluoreszenzebene
- 21: Sammelprismen (führen alle Laser in einen Strahlengang )
- 22: Streulichtebene
- 23: Laser - Durchgangsfenster der Messzelle 17
- 24: Strahlengang Laser
- 25: Befüllöffnung der Messzelle 17
- 26: Messpunkt mit den Fokusebenen für Laserstreulicht und Floureszenzlicht

## Patentansprüche

1. Vorrichtung zur Bestimmung der Fluoreszenz und der Anzahl von Antikörpern auf Exosomen, umfassend:
eine Messzelle (17) zur Aufnahme einer Probenflüssigkeit (18),
eine Videokamera (13) mit Kameraoptik (14),
eine Flüssigkeitslinse (15) mit einstellbarem Fokus,
mehrere verschiedenfarbige Laser (1-5) und für jeden Laser (1-5) ein zugeordnetes Sammelprisma (21), wobei die Vorrichtung dazu ausgebildet ist,
einen Strahl von dem jeweiligen Laser (1-5) über das zugehörige Sammelprisma (21) auf einen für alle Laser (1-5) gleichen Einfallsstrahl (24) zu leiten, der an einer Messposition 1 in die Messzelle (17) einfällt, wobei die Vorrichtung weiter dazu ausgebildet ist, den Einfallsstrahl (24) auf einen Messpunkt (26) im Inneren der Messzelle (17) zu fokussieren, und die Fokussierung des jeweiligen Einfallsstrahls (24) in Wechselwirkung mit der Probenflüssigkeit (18) im Messpunkt (26) als Zentrum eines konvergenten Strahlenbündels nach Durchgang durch die Flüssigkeitslinse (15) in der Videokamera (13) zu registrieren,
eine Wechselvorrichtung (7) mit einem Filter (6) und einem Quarzglas (16) zur Einstellung und Sicherung der gleichen optischen Weglängen für Fluoreszenzlicht und Laserstreulicht in einem konvergenten Strahlengang zwischen der Flüssigkeitslinse (15) und dem Messpunkt (26) in der Messzelle (17),
ein Display (9) mit einem Touchscreen und eine Gesamt-Steuerung (10) mit einem Partikel-Tracking-Programm zur Bedienung der Videokamera (13), **dadurch gekennzeichnet, dass** das Filter (6) ein Multi-Notch-Filter ist, und
dass die Vorrichtung weiter umfasst:
Mittel zum Anfahren verschiedener bestimmter Messpositionen 1-4 durch die Laser (1-5), so dass der jeweilige Einfallsstrahl (24) auf verschiedene Messpunkte (26) in der Messzelle (17) fokussierbar ist,
Mittel zum Speichern der verschiedenen Messpositionen 1-4 und zugehöriger Fokuspunkte der Laser (1-5),
wobei die Vorrichtung dazu ausgebildet ist, die gespeicherten Messpositionen und zugehörigen Fokuspunkte der Laser mit den Lasern anzufahren und dann die Fokussierung des jeweiligen Einfallsstrahls (24) in Wechselwirkung mit der Probenflüssigkeit (18) im jeweiligen Messpunkt (26) als Zentrum eines konvergenten Strahlenbündels nach Durchgang durch die Flüssigkeitslinse (15) in der Videokamera (13) zu registrieren,
und wobei die Vorrichtung so konfiguriert ist, dass die Laser (1-5) beim Verfahren auf die nächste Messposition 1-4 jeweils ausgeschaltet sind.

2. Vorrichtung nach Anspruch 1, **dadurch gekennzeichnet, dass** die Videokamera (13) einen Graphen-basierten Lichtsensor (12) mit einer zugehörigen Steuerung (11) für den Lichtsensor (12) aufweist.

3. Vorrichtung nach einem der Ansprüche 1 oder 2, **dadurch gekennzeichnet, dass** die Vorrichtung schnelle Schritt-Motoren und spielfreie Präzisions- Verfahrschlitten aufweist und die Videokamera (13) ein sCMOS oder ein eCCD ist.

4. Verfahren zur Bestimmung der Fluoreszenz und der Anzahl von Antikörpern auf Exosomen unter Verwendung einer Vorrichtung nach einem der vorhergehenden Ansprüche, umfassend die folgenden Schritte:
- Befüllen der Messzelle (17) mit einer Kalibrierflüssigkeit,
- Anfahren und Erfassen der verschiedenen Messpositionen 1-4 der verwendeten Laser (1-5), Fokussieren der Laser und Speichern der Messpositionen 1-4 und der zugehörigen Fokuspunkte der Laser (1-5), wobei die Messzelle (17) mit der Kalibrierflüssigkeit befüllt ist und sich das Quarzglas (16) im konvergenten Strahlengang zwischen der Flüssigkeitslinse (15) und dem jeweiligen Messpunkt (26) in der Messzelle (17) befindet,
- Tauschen der Kalibrierflüssigkeit gegen eine Probenflüssigkeit mit zu messenden Exosomen und Prüfen auf Blasenfreiheit in der Messzelle (17),
- Durchführen der folgenden Schritte für alle Messpositionen 1-4 nacheinander, wobei die Messzelle (17) mit der Probenflüssigkeit befüllt ist und sich das Multi-Notch-Filter (6) im konvergenten Strahlengang zwischen der Flüssigkeitslinse (15) und dem jeweiligen Messpunkt (26) in der Messzelle (17) befindet:
- Anfahren der jeweiligen Messposition 1-4 mit den Lasern (1-5), wobei die verwendeten Laser nacheinander auf ihren abgespeicherten Fokuspunkten angefahren und beim Beginn einer Analyse eingeschaltet werden,
- Registrieren der Fokussierung des jeweiligen Laserstrahls (24) in Wechselwirkung mit der Probenflüssigkeit (18) im Messpunkt (26) als Zentrum eines konvergenten Strahlenbündels in der Videokamera (13) nach Durchgang durch die Flüssigkeitslinse (15) mit einem Lichtsensor (12) der Videokamera (13),
- Weiterleiten entsprechender Daten an eine Mustererkennung, wobei die Laser (1-5) beim Verfahren auf die nächste Messposition jeweils ausgeschaltet sind.

## Claims

1. A device for determining the fluorescence and the number of antibodies on exosomes, the device comprising:
a measurement cell (17) for accommodating a sample liquid (18),
a video camera (13) with camera optics (14),
a liquid lens (15) with an adjustable focus,
several varicolored lasers (1-5) and, for each laser (1-5), an associated collecting prism (21), wherein the device is configured to guide a beam from the respective laser (1-5) via the associated collecting prism (21) to a single common incident beam (24) for all the lasers (1-5), wherein the incident beam is incident into the measurement cell (17) at a measurement position 1, wherein the device is further configured to focus the incident beam (24) on a measurement point (26) within the measurement cell (17) and to register the focusing of the respective incident beam (24) in interaction with the sample liquid (18) in the measurement point (26) as a center of a convergent beam bundle after passing the liquid lens (15) in the video camera (13),
a change device (7) with a filter (6) and a quartz glass (16) for setting and securing the same optical path lengths for fluorescence light and laser scattered light in a convergent beam path between the liquid lens (15) and the measurement point (26) in the measurement cell (17),
a display (9) having a touchscreen and an overall controller (10) having a particle tracking program for operating the video camera (13),
**characterized in that** the filter (6) is a multi-notch filter, and **in that** the device further comprises:
means for moving to several predetermined measurement positions 1-4 with the lasers (1-5), so that the respective incident beam (24) is focusable on different measurement points (26) in the measurement cell (17);
means for storing the different measurement positions 1-4 and
associated focus points of the lasers (1-5);
wherein the device is configured to move, with the lasers, to the stored measurement positions and the associated focus points of the lasers,
and then to register the focusing of the respective incident beam (24) interacting with the sample liquid (18) in the respective measurement point (26) as center of a convergent beam bundle after passing through the liquid lens (15) in the video camera (13),
and wherein the device is configured in such a manner that the lasers (1-5) are switched off when moving to the next measurement position 1-4.

2. The device according to claim 1, **characterized in that** the video camera (13) has a graphene-based light sensor (12) having an associated controller (11) for the light sensor (12).

3. The device according to any one of claims 1 or 2, **characterized in that** the device has rapid stepping motors and precision movement carriages without play, and the video camera (13) is an sCMOS or an eCCD.

4. A method for determining the fluorescence and the number of antibodies on exosomes by using a device according to any of the preceding claims, the method comprising the following steps:
- filling the measurement cell (17) with a calibration liquid,
- moving to and detecting the different measurement positions 1-4 of the lasers (1-5) that are used, focusing the lasers and storing the measurement positions 1-4 and the associated focus points of the lasers (1-5), wherein the measurement cell (17) is filled with the calibration liquid and the quartz glass (16) is positioned in the convergent beam path between the liquid lens (15) and the respective measurement point (26) within the measurement cell (17),
- replacing the calibration liquid with a sample liquid having exosomes to be measured, and checking the measurement cell (17) for freedom from bubbles,
- performing the following steps for all measurement positions 1-4 one after the other, wherein the measurement cell (17) is filled with the sample liquid and the multi-notch filter (6) is positioned in the convergent beam path between the liquid lens (15) and the respective measurement point (26) within the measurement cell (17),
- moving to the respective measurement position 1-4 with the lasers (1-5), wherein the lasers that are used are moved successively to their stored focus points and are switched on at the beginning of an analysis,
- registering the focusing of the respective laser beam (24) interacting with the sample liquid (18) in the measurement point (26) as center of a convergent beam bundle after passing through the liquid lens (15) in the video camera (13) with a light sensor (12) of the video camera (13),
- forwarding corresponding data to a pattern recognition, wherein the respective lasers (1-5) are switched off when moving to the next measurement position.

## Revendications

1. Dispositif pour déterminer la fluorescence et le nombre d'anticorps sur des exosomes, comprenant :
une cellule de mesure (17) pour recevoir un liquide échantillon (18),
une caméra vidéo (13) avec une optique de caméra (14),
une lentille liquide (15) à mise au point réglable,
plusieurs lasers de différentes couleurs (1-5) et, pour chaque laser (1-5), un prisme collecteur associé (21), le dispositif étant conçu pour diriger un faisceau provenant du laser respectif (1-5) via le prisme collecteur associé (21) vers un faisceau incident identique (24) pour tous les lasers (1-5), qui est incident sur la cellule de mesure (17) en une position de mesure 1, le dispositif étant en outre conçu pour focaliser le faisceau incident (24) sur un point de mesure (26) à l'intérieur de la cellule de mesure (17) et d'enregistrer la focalisation du faisceau incident respectif (24) en interaction avec le liquide échantillon (18) au point de mesure (26) comme centre d'un faisceau convergent après passage à travers la lentille liquide (15) dans la caméra vidéo (13),
un dispositif de commutation (7) avec un filtre (6) et un verre de quartz (16) pour régler et garantir les mêmes longueurs de trajet optique pour la lumière fluorescente et la lumière laser diffusée dans un trajet de faisceau convergent entre la lentille liquide (15) et le point de mesure (26) dans la cellule de mesure (17),
un écran (9) avec un écran tactile et une commande globale (10) avec un programme de suivi de particules pour commander la caméra vidéo (13),
**caractérisé en ce que** le filtre (6) est un filtre Mulit-Notch et **en ce que** le dispositif comprend en outre :
des moyens pour amener les lasers (1-5) à différentes positions de mesure déterminées 1-4, de sorte que le faisceau incident respectif (24) puisse être focalisé sur différents points de mesure (26) dans la cellule de mesure (17),
des moyens pour enregistrer les différentes positions de mesure 1-4 et les points de focalisation associés des lasers (1-5),
le dispositif étant conçu pour atteindre les positions de mesure enregistrées et les points de focalisation associés des lasers à l'aide des lasers, puis pour focaliser le faisceau incident respectif (24) en interaction avec le liquide échantillon (18) au point de mesure respectif (26) en tant que centre d'un faisceau convergent après passage à travers la lentille liquide (15) dans la caméra vidéo (13),
et le dispositif étant configuré de telle sorte que les lasers (1-5) sont respectivement désactivés lors du déplacement vers la position de mesure suivante 1-4.

2. Dispositif selon la revendication 1, **caractérisé en ce que** la caméra vidéo (13) comprend un capteur de lumière à base de graphène (12) avec une commande associée (11) pour le capteur de lumière (12).

3. Dispositif selon l'une des revendications 1 ou 2, **caractérisé en ce que** le dispositif comprend des moteurs pas à pas rapides et des chariots de déplacement de précision sans jeu, et **en ce que** la caméra vidéo (13) est une sCMOS ou une eCCD.

4. Procédé pour déterminer la fluorescence et le nombre d'anticorps sur des exosomes à l'aide d'un dispositif selon l'une des revendications précédentes, comprenant les étapes suivantes :
- remplir la cellule de mesure (17) avec un liquide d'étalonnage,
- démarrer et enregistrer les différentes positions de mesure 1 à 4 des lasers utilisés (1 à 5), focaliser les lasers et enregistrer des positions de mesure 1-4 et des points de focalisation correspondants des lasers (1-5), la cellule de mesure (17) étant remplie du liquide d'étalonnage et le verre de quartz (16) se trouvant dans le trajet convergent des rayons entre la lentille liquide (15) et le point de mesure respectif (26) dans la cellule de mesure (17),
- remplacer le liquide d'étalonnage par un liquide d'échantillon contenant les exosomes à mesurer et vérifier l'absence de bulles dans la cellule de mesure (17),
- exécuter des étapes suivantes pour toutes les positions de mesure 1 à 4 les unes après les autres, la cellule de mesure (17) étant remplie du liquide échantillon et le filtre Mulit-Notch (6) se trouvant dans le trajet convergent des rayons entre la lentille liquide (15) et le point de mesure respectif (26) dans la cellule de mesure (17) :
- amener les lasers (1 à 5) vers la position de mesure respective 1 à 4, les lasers utilisés étant déplacés les uns après les autres vers leurs points de focalisation enregistrés et activés au début d'une analyse,
- enregistrer la focalisation du faisceau laser respectif (24) en interaction avec le liquide échantillon (18) au point de mesure (26) comme centre d'un faisceau convergent dans la caméra vidéo (13) après passage à travers la lentille liquide (15) avec un capteur de lumière (12) de la caméra vidéo (13),
- transmettre les données correspondantes à un système de reconnaissance de motifs, les lasers (1-5) étant désactivés lors du déplacement vers la position de mesure suivante.
